(19) Europäisches Patentamt
European Patent Office
Office européen des brevets

(11) **EP 2 048 184 B1**

(12) **EUROPEAN PATENT SPECIFICATION**

(45) Date of publication and mention
of the grant of the patent:
**05.09.2018 Bulletin 2018/36**

(51) Int Cl.:
*C08J 3/12* *(2006.01)*    *A61F 13/15* *(2006.01)*
*A61F 13/49* *(2006.01)*    *A61F 13/53* *(2006.01)*

(21) Application number: **07791485.1**

(22) Date of filing: **27.07.2007**

(86) International application number:
**PCT/JP2007/064791**

(87) International publication number:
**WO 2008/015980 (07.02.2008 Gazette 2008/06)**

(54) **WATER-ABSORBABLE RESIN PARTICLE, METHOD FOR PRODUCTION THEREOF, AND ABSORBER MATERIAL USING THE SAME**

WASSERABSORBIERBARES HARZTEILCHEN, HERSTELLUNGSVERFAHREN DAFÜR UND ABSORBERMATERIAL UNTER VERWENDUNG DAVON

PARTICULE DE RÉSINE ABSORBANT L'EAU, SON PROCÉDÉ DE PRODUCTION ET MATIÈRE ABSORBANTE QUI L'UTILISE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **04.08.2006 JP 2006213268**

(43) Date of publication of application:
**15.04.2009 Bulletin 2009/16**

(73) Proprietor: **SUMITOMO SEIKA CHEMICALS CO., LTD.**
**Kako-gun, Hyogo 675-0145 (JP)**

(72) Inventors:
• **KONDO, Kimihiko**
**Himeji-shi, Hyogo 672-8076 (JP)**
• **TANIGUCHI, Takayasu**
**Himeji-shi, Hyogo 672-8076 (JP)**
• **NAWATA, Yasuhiro**
**Himeji-shi, Hyogo 672-8076 (JP)**

(74) Representative: **Webster, Jeremy Mark et al**
**Mewburn Ellis LLP**
**City Tower**
**40 Basinghall Street**
**London EC2V 5DE (GB)**

(56) References cited:
EP-A1- 1 457 541    WO-A1-2006/062253
JP-A- 07 088 171    JP-A- 08 157 531
JP-A- 09 124 879    JP-A- 2006 110 545

**Description**

Technical Field

The present invention relates to a water-absorbent resin particle, a method for production thereof, and an absorbent material using the same. More particularly, the present invention relates to a water-absorbent resin particle which is excellent in a particle strength to mechanical impact, and in which the water absorption capacity under pressure even after mechanical impact is hardly reduced, a method for production thereof, and an absorbent material using the same.

Background Art

In recent years, a water-absorbent resin has been widely used in a variety of fields such as hygiene products such as a disposable diaper and a sanitary product, agricultural and horticultural materials such as a water retention agent and a soil conditioner, and industrial materials such as a water blocking material and a dew-catcher. Among these fields, use in hygiene products such as a disposable diaper and a sanitary product has become great utility.

As the water-absorbent resin, for example, a partial neutralization product of a polyacrylic acid, a hydrolysate of a starch-acrylonitrile graft copolymer, a neutralization product of a starch-acrylic acid graft copolymer, a saponification product of a vinyl acetate-acrylic acid ester copolymer and the like are known.

Usually, as the desired property for a water-absorbent resin, there are a high water absorption capacity, an excellent water-absorbing rate, a high gel strength after water absorption and the like. Particularly, as the desired property for a water-absorbent resin used in an absorbent material in hygiene material utility, there are an excellent water absorption capacity under pressure, a suitable particle diameter, small returning of an absorbed substance to the outside of an absorbent material, excellent diffusibility of an absorbed substance into the interior of an absorbent material and the like, in addition to a high water absorption capacity, an excellent water absorbing rate, and a high gel strength after water absorption.

Further, in recent years, with thinning of an absorbent material in hygiene material utility such as a disposable diaper, a sanitary napkin and the like, and speed up in a manufacturing line, since a force applied to a water-absorbent resin particle becomes greater, properties of a high particle strength, and small reduction in performance even after manufacturing of an absorbent material, are becoming necessary.

For example, an absorbent material for a disposable diaper is manufactured by a method of sucking a water-absorbent resin particle and a fibrous pulp on a metal mesh, while mixing them in the air and laminating the mixture, in a facility generally called drum former. Thereafter, an absorbent material is compressed using a roll press in order to enhance a strength, and retain a shape and, particularly in manufacturing of a thin absorbent material, since a material is compressed with a high pressure, and a use amount of a pulp is reduced, a great force is applied to a water-absorbent resin particle, easily causing destruction of a particle.

Further, by proceeding speed-up of an absorbent material manufacturing line in order to enhance productivity, in the drum former, a particle is easily destructed also by collision of a water-absorbent resin particle against a metal mesh and a surrounding supporting plate at a high speed.

Particularly, in a recent water-absorbent resin particle, since in order to improve the water-absorbing performance, for example, a crosslinking density of a surface layer of a water-absorbent resin particle is increased, when destruction of a water-absorbent resin particle is caused, the interior of a particle having a low crosslinking density is exposed on a surface, easily causing remarkable reduction in performance.

Accordingly, a water-absorbent resin particle having a high particle strength against mechanical impact, and water-absorbing performance of which is not reduced, is demanded.

As such the water-absorbent resin particle, for example, a water-absorbent resin particle having improved brittlement of a particle, and having a water content of 3 to 9%, and a breakage stress of a particle of 30 N/m$^2$ or more, is known (see Patent Literature 1). However, for using in a thin absorbent material produced at a high speed, this water-absorbent resin particle has an insufficient particle strength, performance is reduced by destruction with collision, and performance of an absorbent material may be reduced.

Patent Literature 2 describes a water absorbing agent comprising a cross-linked polymer having a moisture content of no more than 12%.

Patent Literature 3 describes a method for surface-treatment of a water absorbent resin having a water content of no more than 6.9%.

Patent Literature 1: JP-A No. 9-124879
Patent Literature 2: EP 1 457 541
Patent Literature 3: WO 2006/062253

Disclosure of the Invention

Problems to be Solved by the Invention

An object of the present invention is to provide a water-absorbent resin particle which is excellent in powder flowability at a high moisture content, excellent in a particle strength, and high in a particle diameter retaining rate and a retaining rate of water absorption capacity under pressure even after mechanical impact, a method for production thereof, and an absorbent material using the same.

Means to Solve the Problems

That is, the present invention relates to a method for production of water-absorbent resin particles comprising polymerizing a water-soluble ethylenic unsaturated monomer using a water-soluble radical polymerization initiator, if necessary, in the presence of a crosslinking agent, to obtain water-absorbent resin particle precursors, adding a post-crosslinking agent to crosslink a surface layer of the particle precursors, adding amorphous silica particles in an amount of 0.01 to 2 parts by mass per 100 parts per mass of water-absorbent resin particles, and adjusting a moisture content of the resulting water-absorbent resin particles to 12 to 20%, whereby the obtained water-absorbent resin particles have a retaining rate of particle diameter after a particle collision test of 90% or more.
The present invention also relates to water-absorbent resin particles as obtainable by the method.
The present invention further relates to an absorbent material consisting of the water-absorbent resin particles, a hydrophilic fiber and a water-permeable sheet.

Effects of the invention

Since the water-absorbent resin particle of the present invention is a water-absorbent resin particle which is excellent in powder flowability at a high moisture content, excellent in a particle strength, high in a particle diameter retaining rate and a retaining rate of water absorption capacity under pressure even after mechanical impact, and is excellent in a water absorbing rate, it is suitable for use in a thin absorbent material produced at a high speed, and the resulting thin absorbent material and absorbent product have the characteristic that absorbability of a liquid to be absorbed is excellent, and leakage is small.

Brief Description of the Drawings

Fig. 1 is a schematic view showing an outline construction of a device for measuring the water absorption capacity under pressure.
Fig. 2 is a schematic view showing an outline construction of a device for carrying out a collision test.

Explanation of symbols

X Measuring device
1 Burette part
10 Burette
11 Air introducing tube
12 Cock
13 Cock
14 Rubber plug
2 Conduit
3 Measurement stand
4 Measuring part
40 Cylinder
41 Nylon mesh
42 Weight
5 Water-absorbent resin particle
Y Collision test device

101 Hopper
102 Pressurized air introducing tube
103 Injection nozzle
104 Impinging plate
105 Flowmeter
106 Water-absorbent resin particle

Best Mode for Carrying Out the Invention

It is preferable that the water-absorbent resin particle of the present invention is a water-absorbent resin particle obtained by polymerizing a water-soluble ethylenic unsaturated monomer using a water-soluble radical polymerization initiator, if necessary, in the presence of a crosslinking agent, to obtain the water-absorbent resin particle precursor, adding a post-crosslinking agent to crosslink a surface layer of the particle, and adding an amorphous silica particle.

A moisture content of the water-absorbent resin particle of the present invention is 12 to 20%, preferably 12 to 18%. When a moisture content of the water-absorbent resin particle is less than 12%, there is a possibility that destruction of the particle by mechanical impact is easily caused, and a sufficient strength is not obtained. On the other hand, when a moisture content of the water-absorbent resin particle is more than 20%, there is a possibility that powder flowability of the water-absorbent resin is deteriorated, and handling becomes difficult.

A moisture content of the water-absorbent resin particle is a value measured according to the measuring method described later in "(1) Moisture content".

A particle diameter retaining rate after a particle collision test of the water-absorbent resin particle of the present invention is 90% or more, preferably 92% or more, more preferably 94% or more. When the particle diameter retaining rate is less than 90%, the water absorption capacity under pressure may be deteriorated by destruction of the surface crosslinked layer.

The particle diameter retaining rate after a particle collision test of the water-absorbent resin particle is a value measured according to the measuring method described later in "(6) Particle diameter retaining rate after particle collision test".

A retaining rate of water absorption capacity under pressure after a particle collision test of the water-absorbent resin particle of the present invention is preferably 60% or more, more preferably 65% or more. When the retaining rate of water absorption capacity under pressure is less than 60%, the absorbent material performance may be deteriorated.

The retaining rate of water absorption capacity under pressure after a particle collision test of the water-absorbent resin particle is a value measured according to the measuring method described later in "(7) Retaining rate of water absorption capacity under pressure after particle collision test".

A method for production of the water-absorbent resin particle of the present invention is not particularly limited, but examples include a method of polymerizing a water-soluble ethylenic unsaturated monomer using a water-soluble radical polymerization initiator, if necessary, in the presence of a crosslinking agent, to obtain a water-absorbent resin particle precursor, adding a post-crosslinking agent to crosslink a surface layer of the particle, adding an amorphous silica particle, and adjusting a moisture content of the resulting water-absorbent resin particle to 12 to 20%. A polymerization method is not particularly limited, but examples include an aqueous solution polymerization method, a reversed-phase suspension polymerization method and the like, which are a representative polymerization method. Among them, from a viewpoint that powder flowability is excellent at a high moisture content, a reversed-phase suspension polymerization method of polymerizing the water-soluble ethylenic unsaturated monomer using a water-soluble radical polymerization initiator in an organic solvent with a surfactant added thereto is preferably used.

Examples of the water-soluble ethylenic unsaturated monomer include (meth)acrylic acid ["(meth)acry" means "acry" or "methacry"; the same hereinafter], 2-(meth)acrylamide-2-methylpropanesulfonic acid or a salt thereof; nonionic monomers such as (meth)acrylamide, N,N-dimethylacrylamide, 2-hydroxyethyl (meth)acrylate, N-methylol(meth)acrylamide etc.; amino group-containing unsaturated monomers such as diethylaminoethyl (meth)acrylate, diethylaminopropyl (meth)acrylate etc., or quaternarized products thereof. These may be used alone, or may be used by mixing two or more kinds of them.

And, when the monomer has an acid group, examples of an alkali compound used for neutralizing it include compounds of lithium, sodium, potassium, ammonium and the like and, among them, sodium hydroxide, and potassium hydroxide are preferable from a viewpoint of economy and performance.

And, when the monomer having an acid group is neutralized, a neutralization degree of it is preferably 30 to 90 mol% of an acid group of a water-soluble ethylenic unsaturated monomer. When the neutralization degree is lower than 30%, the acid group is ionized with difficulty, and the water absorption capacity is lowered, being not preferable. When the neutralization degree is higher than 90%, in the case of use as hygiene materials, there is a possibility that a problem arises in safety, being not preferable.

Preferable examples of the water-soluble ethylenic unsaturated monomer include (meth)acrylic acid or a salt thereof from a viewpoint of industrial easy availability.

A concentration of an aqueous solution of the water-soluble ethylenic unsaturated monomer is preferably from 20% by mass to a saturated concentration.

Examples of the crosslinking agent which is added to the water-soluble ethylenic unsaturated monomer, if necessary, include di- or tri-(meth)acrylic acid esters of polyols such as ethylene glycol, propylene glycol, trimethylolpropane, glycerin, polyoxyethylene glycol, polyoxypropylene glycol, polyglycerin etc.; unsaturated polyesters obtained by reacting the polyols and unsaturated acids such as maleic acid, fumaric acid etc.; bisacrylamides such as N,N'-methylenebisacrylamide etc.; di- or tri-(meth)acrylic acid esters obtained by reacting polyepoxides and (meth)acrylic acid; di(meth)acrylic acid carbamyl esters obtained by reacting polyisocyanates such as tolylene diisocyanate, hexamethylene diisocyanate etc. and hydroxyethyl (meth)acrylate; compounds having two or more polymerizable unsaturated groups such as allylated starch, allylated cellulose, diallyl phthalate, N,N',N"-triallyl isocyanate, divinylbenzene etc.; diglycidyl ether compounds such as (poly)ethylene glycol diglycidyl ether ["(poly)" means the case where there is no prefix of "poly", and the case where there is the prefix; the same hereinafter], (poly)propylene glycol diglycidyl ether, (poly)glycerin diglycidyl ether etc.; haloepoxy compounds such as epichlorohydrin, epibromohydrin, $\alpha$-methylepichlorohydrin etc.; compounds having two or more reactive functional groups such as isocyanate compounds such as 2,4-tolylene diisocyanate, hexamethylene diisocyanate etc. These may be used alone, or may be used by mixing two or more kinds of them.

An addition amount of the crosslinking agent is preferably 3 parts by mass or less, more preferably 0.001 to 1 part by mass based on 100 parts by mass of the water-soluble ethylenic unsaturated monomer. When the addition amount is more than 3 parts by mass, water absorbability of the resulting polymer is reduced, being not preferable.

Examples of the water-soluble radical polymerization initiator used in the present invention include persulfates such as potassium persulfate, ammonium persulfate, sodium persulfate etc.; azo compounds such as 2,2'-azobis(2-amidinopropane) dihydrochloride, azobis(cyanovaleric acid) etc. These may be used alone, or may be used by mixing two or more kinds of them.

Alternatively, by using the water-soluble radical polymerization initiator together with sulfite, L-ascorbic acid, ferrous sulfate or the like, it may be also used as a redox polymerization initiator.

Among them, potassium persulfate, ammonium persulfate and sodium persulfate are preferable from a viewpoint of easy availability and better storage stability.

A use amount of the water-soluble radical polymerization initiator is preferably 0.001 to 1 part by mass, more preferably 0.01 to 0.5 part by mass based on 100 parts by mass of the water-soluble ethylenic unsaturated monomer. When the amount is less than 0.001 part by mass, a polymerization reaction does not sufficiently proceed and, when the amount is more than 1 part by mass, a polymerization reaction becomes rapid, and the reaction can not be controlled, being not preferable.

In the present invention, after the water-absorbent resin particle precursor is obtained, a post-crosslinking agent having two or more functional groups having the reactivity with functional groups of the water-soluble ethylenic unsaturated monomer is added to crosslink a surface layer of the particle precursor.

Examples of the post-crosslinking agent used include polyols such as ethylene glycol, propylene glycol, 1,4-butanediol, trimethylolpropane, glycerin, polyoxyethylene glycol, polyoxypropylene glycol, polyglycerin etc.; diglycidyl ether compounds such as (poly)ethylene glycol diglycidyl ether, (poly)propylene glycol diglycidyl ether, (poly)glycerin diglycidyl ether etc.; haloepoxy compounds such as epichlorohydrin, epibromohydrin, $\alpha$-methyl epichlorohydrin etc.; compounds having two or more reactive functional groups such as isocyanate compounds such as 2,4-tolylene diisocyanate, hexamethylene diisocyanate etc.; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, 3-butyl-3-oxetaneethanol etc., oxazoline compounds such as 1,2-ethylenebisoxazoline etc., carbonate compounds such as ethylene carbonate etc., hydroxyalkylamide compounds such as bis[N,N-di($\beta$-hydroxyethyl)]adipamide. These may be used alone, or may be used by mixing two or more kinds of them.

Among them, from a viewpoint of the excellent reactivity, ethylene glycol diglycidyl ether, propylene glycol diglycidyl ether, glycerin diglycidyl ether, polyethylene glycol diglycidyl ether, polypropylene glycol diglycidyl ether, and polyglycerin diglycidyl ether are preferable.

An addition amount of the post-crosslinking agent is preferably 0.01 to 5 parts by mass, more preferably 0.03 to 3 parts by mass based on a total amount of 100 parts by mass of the water-soluble ethylenic unsaturated monomer subjected to polymerization. When the addition amount of the post-crosslinking agent is less than 0.01 part by mass, a gel strength of the resulting water-absorbent resin particle becomes weak and, when the addition amount is more than 5 parts by mass, a crosslinking density becomes excessive, and sufficient water absorbability is not exhibited, being not preferable.

A time of addition of the post-crosslinking agent to the water-absorbent resin particle precursor is any time as far as it is after a polymerization reaction, being not limiting. Mixing of the water-absorbent resin particle precursor and the post-crosslinking agent is performed preferably in the presence of 200 parts by mass or less of water, more preferably in the presence of water in a range of 1 to 100 parts by mass, further preferably in the presence of water in a range of 5 to 50 parts by mass, based on 100 parts by mass of the water-absorbent resin particle precursor. Like this, by adjusting an amount of water at addition of the crosslinking agent, a surface layer of the water-absorbent resin particle can be more suitably crosslinked, and the excellent water absorption capacity under pressure can be attained.

The thus obtained water-absorbent resin particle is dried by removing water and an organic solvent in a drying step. And, the drying step may be conducted under reduced pressure.

In the present invention, a method of adjusting a moisture content of the water-absorbent resin particle is not particularly limited as far as it is a method by which a final moisture content is in a range of 12 to 20%. Examples include a method of controlling a moisture content of the final water-absorbent resin particle in a range of 12 to 20% by regulating a drying temperature and time in a stage of drying a hydrous water-absorbent resin particle after polymerization, a method of controlling a moisture content of the final water-absorbent resin particle in a range of 12 to 20% by moistening a water-absorbent resin particle which has been dried to a moisture content of less than 12%, under stirring, and the like.

A mass median particle diameter of the thus obtained water-absorbent resin particle of the present invention is preferably 200 to 500 μm, more preferably 250 to 400 μm. When the mass median particle diameter is less than 200 μm, a gap between particles is small, permeability of an absorbed liquid is reduced, and gel blocking is easily caused, being not preferable. On the other hand, when the mass median particle diameter is more than 500 μm, a water absorbing rate becomes too slow and, when used in an absorbent material, liquid leakage is easily caused, being not preferable.

And, the mass median particle diameter of the water-absorbent resin particle is a value measured according to the measuring method described later in "(5) Mass median particle diameter".

In order to improve powder flowability at a high moisture content, an amorphous silica particle is added to and mixed into the water-absorbent resin particle of the present invention. An median particle diameter of the amorphous silica particle, in order to obtain effective powder flowability at addition of a small amount, is preferably 20 μm or less, more preferably 15 μm or less. A specific surface area of the amorphous silica particle is preferably 50 to 500 m$^2$/g, more preferably 100 to 300 m$^2$/g. And, the amorphous silica particle may be produced by either of a wet method or a dry method, and may be hydrophobicized by chemical treatment with octylsilane or the like, or surface treatment with a dimethylsilicone oil or the like. Examples of the amorphous silica particle include Tokuseal NP manufactured by Tokuyama Co., Ltd. (median particle diameter 11 μm, specific surface area 195 m$^2$/g), Fineseal T-32 (median particle diameter 1.5 μm, specific surface area 202 m$^2$/g), and the like.

An addition amount of the amorphous silica particle is preferably 0.01 to 2 parts by mass, more preferably 0.1 to 1.5 parts by mass, further preferably 0.3 to 1 part by mass, further more preferably 0.5 to 0.7 part by mass based on 100 parts by mass of the water-absorbent resin particle. When an addition amount of the amorphous silica particle is less than 0.01 part by mass, the effect of improving powder flowability is low and, when the addition amount is more than 2 parts by mass, a dusting degree is increased, being not preferable. The water-absorbent resin particle of the present invention has a high moisture content, and the amorphous silica particle can be effectively adhered to a particle surface.

In addition, by adding the amorphous silica particle to the water-absorbent resin particle, a gap is generated between particles, and permeability of an absorbed liquid is improved.

An absorbent material using the water-absorbent resin particle of the present invention will be explained below. The absorbent material of the present invention consists of a water-absorbent resin particle, a hydrophilic fiber and a water-permeable sheet. And, the absorbent material of the present invention is preferably used in disposable absorbent products such as a disposable diaper, an incontinence pad, a sanitary napkin, a pet sheet and the like.

Examples of the hydrophilic fiber used in the absorbent material include cellulose fibers such as a cotton-like pulp, a mechanical pulp, a chemical pulp and the like obtained from a timber, artificial cellulose fibers such as rayon, acetate and the like, and the like, and the present invention is not limited to such the exemplification.

Examples of a structure of the absorbent material of the present invention include a structure where a laminate in which a water-absorbent resin particle and a hydrophilic fiber are blended, or a laminate in which a water-absorbent resin particle is scattered between hydrophilic fibers laminated into a sheet, is wrapped with a tissue paper or a water-permeable sheet such as a non-woven fabric, but the present invention is not limited to such the exemplification.

A ratio of the water-absorbent resin particle and the hydrophilic fiber in the absorbent material is preferably a mass ratio of 30:70 to 80:20, more preferably a mass ratio of 40:60 to 60:40.

A density of the absorbent material is preferably 0.1 to 0.5 g/cm$^3$, more preferably 0.2 to 0.4 g/cm$^3$.

In addition, an absorbent product using the absorbent material of the present invention has a structure in which the absorbent material is retained between a liquid-permeable sheet with which an aqueous liquid is permeable (top sheet), and a liquid-impermeable sheet with which an aqueous liquid is not permeable (back sheet). The liquid-permeable sheet is disposed on a side contacting with a body, and the liquid-impermeable sheet is disposed on a side opposite to a side contacting with a body.

Examples of the liquid-permeable sheet include a non-woven fabric consisting of a synthetic resin such as polyethylene, polypropylene, polyester, polyamide and the like, a porous synthetic resin sheet and the like.

Examples of the liquid-impermeable sheet include a film consisting of a synthetic resin such as polyethylene, polypropylene, polyvinyl chloride and the like, a sheet consisting of a composite material of these synthetic resins and a non-woven fabric.

Examples

The following Examples and Comparative Examples illustrate the present invention, but the present invention is not limited by these Examples.

Preparation Example 1

Into an Erlenmeyer flask of an internal volume of 500 ml placed 92 g of a 80 mass% aqueous acrylic acid solution, and 154.1 g of a 20.0 mass% aqueous sodium hydroxide solution was added dropwise while ice-cooling, to neutralize acrylic acid, thereby, an aqueous acrylic acid partial neutralized salt solution was prepared. To the resulting aqueous acrylic acid partial neutralized salt solution were added 9.2 mg of N,N'-methylenebisacrylamide as a crosslinking agent, and 0.11 g of potassium persulfate as a water-soluble radical polymerization initiator, and this was used as an aqueous monomer solution.

Separately, a five-necked cylinder-type round-bottom flask of an internal volume of 2 liter equipped with a stirrer, a double-paddle blade, a refluxing condenser, an addition funnel and a nitrogen gas introducing tube was charged with 340 g of n-heptane, and 0.92 g of sugar stearic acid ester (trade name of Mitsubishi-Kagaku Foods Corporation; Ryoto Sugar Ester S-370) as a surfactant to dissolve them in n-heptane, the aqueous monomer solution for polymerization was added, and this was suspended under stirring while retaining at 35°C. Thereafter, the interior of the system was replaced with nitrogen, and a temperature was raised using a water bath at 70°C, followed by reversed-phase suspension polymerization.

Then, separately, 128.8 g of a 80 mass% aqueous acrylic acid solution was placed into an Erlenmeyer flask of an internal volume of 500 ml, 173.8 g of a 24.7 mass% aqueous sodium hydroxide solution was added dropwise while ice-cooling, to neutralize acrylic acid, thereby, an aqueous acrylic acid partial neutralized salt solution was prepared. To the resulting aqueous acrylic acid partial neutralized salt solution were added 12.9 mg of N,N'-methylenebisacrylamide as a crosslinking agent, and 0.16 g of potassium persulfate as a water-soluble radical polymerization initiator, and this was used as an aqueous monomer solution for a second-stage reversed-phase suspension polymerization.

After completion of the first-stage reversed-phase suspension polymerization, the polymerization slurry was cooled, the aqueous monomer solution for a second-stage polymerization was added dropwise to the system, and a mixture was stirred for 30 minutes while retaining at 23°C. Thereafter, the interior of the system was replaced with nitrogen, and a temperature was raised using a water bath at 70°C, followed by second-stage reversed-phase suspension polymerization. After completion of polymerization, the reaction was heated with an oil bath at 120°C, 266 g of water was removed to the outside of the system by azeotropic distillation, 8.83 g of a 2 mass% aqueous ethylene glycol diglycidyl ether solution was added, and post-crosslinking treatment was conducted while retaining at 80°C for 2 hours. Further, water and n-heptane were removed by distillation, followed by drying to obtain 227.2 g of a water-absorbent resin particle having a mass median particle diameter of 360 $\mu$m and a moisture content of 5%.

Reference Example 1

To 200 g of the water-absorbent resin particle obtained as in Preparation Example 1 was added 1 g of an amorphous silica particle (Tokuseal NP, manufactured by Tokuyama Co., Ltd.), the materials were mixed, and placed into a separable flask of an internal volume of 2 liter, the interior of the separable flask was humidified with a humidifier (hybrid humidifier, manufactured by Toyotomi Co., Ltd.) at a water addition amount of 0.4 L/h at room temperature for 20 minutes under stirring, to obtain a water-absorbent resin having a moisture content of 11%.

Example 2

To 200 g of the water-absorbent resin particle obtained as in Preparation Example 1 was added 1 g of an amorphous silica particle (Fineseal T-32, manufactured by Tokuyama Co., Ltd.), materials were mixed, and placed into a separable flask of an internal volume of 2 liter, and the interior of the separable flask was humidified with a humidifier (hybrid humidifier, manufactured by Toyotomi Co., Ltd.) at a water addition amount of 0.4 L/h at room temperature for 30 minutes while stirring, to obtain a water-absorbent resin having a moisture content of 13%.

Example 3

To 200 g of the water-absorbent resin particle obtained as in Preparation Example 1 was added 2 g of an amorphous silica particle (Tokuseal NP, manufactured by Tokuyama Co., Ltd.), materials were mixed, and placed into a separable flask of an internal volume of 2 liter, and the interior of the separable flask was humidified with a humidifier (hybrid humidifier, manufactured by Toyotomi Co., Ltd.) at a water addition amount of 0.4 L/h at room temperature for 45 minutes while stirring, to obtain a water-absorbent resin having a moisture content of 17%.

Example 4

According to the same manner as that of Preparation Example 1, first-stage and second-stage reversed-phase suspension polymerization was conducted. After completion of polymerization, this was heated with an oil bath at 120°C, 255 g of water was removed to the outside of the system by azeotropic distillation, 4.43 g of a 2 mass% aqueous ethylene glycol diglycidyl ether solution was added, this was retained at 80°C for 2 hours to perform crosslinking treatment. Further, water and n-heptane were removed by distillation to dry the polymer, and 1.5 g of an amorphous silica particle (Tokuseal NP, manufactured by Tokuyama Co., Ltd.) was added, followed by mixing to obtain 233.5 g of a water-absorbent resin particle having a mass median particle diameter of 370 μm and a moisture content of 13%.

Comparative Example 1

According to the same manner as that of Preparation Example 1, a water-absorbent resin particle having a moisture content of 5% was obtained.

Comparative Example 2

Into a separable flask of an internal volume of 2 liter was placed 200 g of the water-absorbent resin particle obtained as in Preparation Example 1, and the interior of the separable flask was humidified with a humidifier (hybrid humidifier, manufactured by Toyotomi Co., Ltd.) at a water addition amount of 0.4 L/h at room temperature for 15 minutes while stirring, to obtain a water-absorbent resin having a moisture content of 8%.

Comparative Example 3

Into a separable flask of an internal volume of 2 liter was placed 200 g of the water-absorbent resin particle obtained as in Preparation Example 1, and the interior of the separable flask was humidified with a humidifier (hybrid humidifier, manufactured by Toyotomi Co., Ltd.) at a water addition amount of 0.4 L/h at room temperature for 45 minutes while stirring, to obtain a water-absorbent resin having a moisture content of 17%. This water-absorbent resin had stickiness, and was inferior in flowability of powder, and measurement of a particle size distribution, a collision test described later, and formation of an absorbent core were impossible.

Comparative Example 4

Into a separable flask of an internal volume of 2 liter was placed 200 g of the water-absorbent resin particle obtained as in Preparation Example 1, and the interior of the separable flask was humidified with a humidifier (hybrid humidifier, manufactured by Toyotomi Co., Ltd.) at a water addition amount of 0.4 L/h at room temperature for 60 minutes while stirring, to obtain a water-absorbent resin having a moisture content of 23%. This water-absorbent resin had stickiness, and was inferior in flowability of powder, and measurement of a particle size distribution, a collision test described later, and formation of an absorbent core were impossible.

Physical properties of water-absorbent resin particles obtained in each Example and each Comparative Example were evaluated by the following methods. Results are shown in Table 1 and Table 2.

(1) Moisture content

Into an aluminum foil case (No. 8) which had been adjusted to a constant weight (Wa(g)) in advance was taken 2 g of a water-absorbent resin particle, and the resin particle was precisely weighed (Wd(g)). The sample was dried for 2 hours with a hot air dryer (manufactured by ADVANTEC) having an internal temperature set at 105°C, and allowed to cool in a desiccator, and a mass We(g) after drying was measured. From the following equation, a moisture content of the water-absorbent resin particle was calculated.

$$\text{Moisture content (\%)} = [(Wd-Wa)-(We-Wa)]/(Wd-Wa) \times 100$$

(2) Physiological saline water retention capacity

Into a cotton bag (Cotton Broad No.60, transverse 100 mm × longitudinal 200 mm) was placed 2.00 g of a water-absorbent resin particle, and this was placed into a 500 mL beaker. Into this cotton bag was poured 500 g of a physiological saline, an opening was tied with a rubber band, and this was allowed to stand for 1 hour. Thereafter, the cotton bag was dehydrated for 1 minute using a dehydrater of a centrifugal force of 167G (Model H-122, manufactured by Kokusan-enshinki Co., Ltd.), and a mass Wa(g) of the cotton bag containing a swollen gel after dehydration was measured. The same procedure was performed without adding the water-absorbent resin, an empty mass Wb(g) at wetting of the cotton bag was measured, and the physiological saline water retention capacity was calculated by the following equation.

$$\text{Physiological saline water retention capacity (g/g)} =$$

$$[Wa-Wb](g)/2.00(g)$$

(3) Water absorbing rate

Into a 100 ml beaker was placed $50\pm0.1$ g of a physiological saline at a temperature of $25\pm0.2°C$, followed by adjustment to 600 rpm using a magnetic stirrer bar (8 mmΦ × 30 mm). Then, $2.0\pm0.002$ g of a water-absorbent resin was rapidly added to the beaker and, when addition was completed, a stopwatch was started at the same time. A time (sec) until the water-absorbent resin absorbs a physiological saline, and a vortex disappears was measured with the stopwatch, and this was adopted as the water-absorbing rate.

(4) Water absorption capacity under pressure

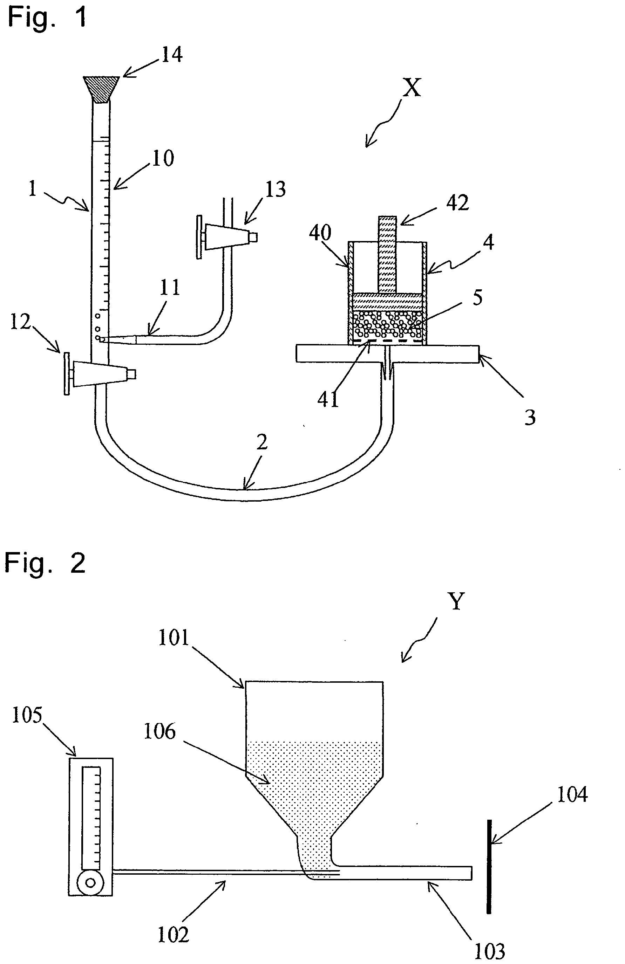

Water absorption capacity under pressure of the water-absorbent resin particle was measured using a measuring device X outlined in Fig.1.
The measuring device X shown in Fig.1 consists of a burette part 1, a conduit 2, a measuring stand 3, and a measuring part 4 placed on the measuring stand 3. The burette part 1 is such that a rubber plug 14 is connected to an upper part of a burette 10, and a suction air introducing tube 11 and a cock 12 are connected to a lower part thereof and, further, the suction air introducing tube 11 has a cock 13 at its tip. The conduit 2 is attached to from the burette part 1 to the measuring stand 3, and a diameter of the conduit 2 is 6 mm. There is a hole of a diameter 2 mm at a central part of the measuring stand 3, and the conduit 2 is connected thereto. The measuring part 4 has a cylinder 40, a nylon mesh 41 attached to a bottom of this cylinder 40, and a weight 42. An internal diameter of the cylinder 40 is 20 mm. The nylon mesh 41 is formed into 200 mesh (aperture 75 μm). And, a predetermined amount of the water-absorbent resin particle 5 is uniformly scattered on the nylon mesh 41. The weight 42 has a diameter of 19 mm, and a mass of 59.8 g. This weight is placed on the water-absorbent resin particle 5, and a load of 2.07 kPa can be applied to the water-absorbent resin particle 5.
In the measuring device X having such the construction, first, the cock 12 and the cock 13 of the burette part 1 are closed, a 0.9 mass% saline regulated at 25°C is placed through an upper part of the burette 10, the upper part of the burette is stopped with the rubber plug 14, and the cock 12 and the cock 13 of the burette part 1 are opened.
Then, a height of the measuring stand 3 is adjusted so that a meniscus of a 0.9 mass% saline exiting from the conduit at a central part of the measuring stand 3, and an upper side of the measuring stand 3 become the same height.
Separately, 0.10 g of the water-absorbent resin particle 5 is uniformly scattered on the nylon mesh 41 of the cylinder 40, and a weight 42 is placed on this water-absorbent resin particle 5. The measuring part 4 is such that a central part thereof is consistent with the conduit at the central part of the measuring stand 3.
Reduction in an amount of the 0.9 mass% saline (i.e. amount of 0.9 mass% saline absorbed by water-absorbent resin particle 5) Wc(ml) is read continuously from the timepoint that the water-absorbent resin particle 5 began to absorb water. The water absorption capacity under pressure of the water-absorbent resin particle 5 after 60 minutes from water absorption initiation was obtained by the following equation.

$$\text{Water absorption capacity under pressure (ml/g) = Wc} \div$$

$$0.10$$

(5) Mass median particle diameter

JIS standard sieves were combined in an order from an upper part of an aperture 500 $\mu$m (30 mesh), an aperture 355 $\mu$m (42 mesh), an aperture 300 $\mu$m (50 mesh), an aperture 250 $\mu$m (60 mesh), an aperture 150 $\mu$m (100 mesh), an aperture 75 $\mu$m (200 mesh), and a saucer, about 100 g of the water-absorbent resin was placed into an uppermost sieve, and this was shaken for 20 minutes using a Rotap shaker.

Then, a mass of the water-absorbent resin remaining on each sieve was calculated as a mass percentage relative to a total amount, and the mass was accumulated in an order from a larger particle diameter, thereby, a relationship between an aperture of a sieve and an accumulated value of a mass percentage remaining on a sieve was plotted on a logarithmic probability paper. Plots on the probability paper were connected with a straight line, thereby, a particle diameter corresponding to an accumulated mass percentage of 50 mass% was adopted as a mass median particle diameter.

(6) Particle diameter retaining rate after particle collision test

A particle diameter retaining rate in a particle collision test of the water-absorbent resin particle was obtained by measuring a particle diameter distribution when the water-absorbent resin particle was collided against an impinging plate, using a test device Y outlined in Fig.2.

The test device Y shown in Fig.2 consists of a hopper 1, a pressurized air introducing tube 2, an injection nozzle 3, an impinging plate 4, and a flowmeter 5. The pressurized air introducing tube 2 is introduced into the interior of the hopper 1, and the injection nozzle 3 is connected to the hopper 1. An external diameter of the pressurized air introducing tube 2 is 3.7 mm, and an internal diameter thereof is 2.5 mm, an external diameter of the injection nozzle 3 is 8 mm, an internal diameter thereof is 6 mm, and a length thereof is 300 mm. A material of the impinging plate 4 is SUS304, a thickness thereof is 4 mm, and a distance between a tip of the injection nozzle 3 and the impinging plate 4 is fixed at 10 mm. The flowmeter 5 is adjusted so that a flow rate of the pressurized air is 50 m/s at a tip of the injection nozzle 3.

In the test device Y having such the construction, first, 100 g of the water-absorbent resin particle 6, a mass median particle diameter (A1) before collision of which has been measured in advance, is placed into the hopper 1. Then, the pressurized air having an adjusted pressure is introduced through the pressurized air introducing tube 2, and the water-absorbent resin particle 6 is injected to the impinging plate 4 through the injection nozzle 3. The water-absorbent resin particle after injection and collision of a total amount is collected, and a particle diameter distribution is measured, thereby, a mass median particle diameter (A2) after collision is obtained.

Using the resulting measured value, a particle diameter retaining rate after a particle collision test was obtained by the following equation.

$$\text{Particle diameter retaining rate after particle collision}$$

$$\text{test (\%) = [A2} \div \text{A1]} \times 100$$

(7) Retaining rate of water absorption capacity under pressure after particle collision test

According to the method described in the "(6) Particle diameter retaining rate after particle collision test", 100 g of the water-absorbent resin particle was subjected to a particle collision test.

Using the recovered water-absorbent resin particle, the water absorption capacity under pressure was measured according to the aforementioned method, and the water absorption capacity under pressure (B2) after a particle collision test was obtained.

From the water absorption capacity under pressure (B1) measured before a particle collision test in advance, and the water absorption capacity under pressure (B2) after a particle collision test, a retaining rate of water absorption capacity under pressure after a particle collision test was obtained by the following equation.

```
Retaining rate of water absorption capacity under
pressure after particle collision test (%) = [B2 ÷ B1] ×
100
```

Table 1

|  | Moisture content (%) | Physiologi cal saline water retention capacity (g/g) | Water absorbing rate (sec) | Water absorption capacity under pressure (g/g) | Mass median particle diameter ($\mu$m) |
|---|---|---|---|---|---|
| Example 1* | 11 | 34 | 41 | 23 | 365 |
| Example 2 | 13 | 33 | 40 | 22 | 365 |
| Example 3 | 17 | 33 | 34 | 22 | 372 |
| Example 4 | 13 | 33 | 38 | 22 | 370 |
| Comparative Example 1 | 5 | 37 | 48 | 23 | 363 |
| Comparative Example 2 | 8 | 35 | 44 | 23 | 366 |
| Comparative Example 3 | 17 | 33 | 38 | 22 | Unmeasurable |
| Comparative Example 4 | 23 | 30 | 30 | 20 | Unmeasurable |
| *Reference example | | | | | |

Table 2

|  | Mass median particle diameter after particle collision test ($\mu$m) | Particle diameter retaining rate after particle collision test (%) | Water absorption capacity under pressure after particle collision test (g/g) | Retaining rate of water absorption capacity under pressure after particle collision test (%) |
|---|---|---|---|---|
| Example 1* | 336 | 92 | 14 | 63 |
| Example 2 | 343 | 94 | 14 | 65 |
| Example 3 | 357 | 96 | 17 | 77 |
| Example 4 | 348 | 94 | 15 | 66 |
| Comparative Example 1 | 290 | 80 | 10 | 43 |
| Comparative Example 2 | 307 | 84 | 11 | 48 |
| Comparative Example 3 | Collision test was impossible | | | |
| Comparative Example 4 | Collision test was impossible | | | |
| *Reference example | | | | |

From the results shown in Table 2, it is seen that all of the water-absorbent resin particles obtained in respective Examples are excellent in a particle diameter retaining rate after a particle collision test, and a retaining rate of water absorption capacity under pressure.

Reference Example 5

Using 8 g of the water-absorbent resin particle after a particle collision test of Reference Example 1 and 12 g of a ground pulp (Rayfloc manufactured by Rayoneir), they were uniformly mixed by air sheet making to make a sheet-like absorbent material core of a size of 42 cm $\times$ 12 cm.

Then, an upper side and a lower side of the absorbent material core were compressed using a roll press in the state where they were held with a tissue paper having a basis weight of 16 g/m$^2$, to make an absorbent material having a density of 0.2 g/cm$^3$.

Further, a top sheet of a polyethylene non-woven fabric (manufactured by Rengo Co., Ltd.) having a basis weight of 22 g/m$^2$ was placed on an upper side of the absorbent material, which was used as an absorbent material for a test.

Example 6

According to the same manner as that of Reference Example 5 except that the water-absorbent resin particle after a particle collision test of Example 2 was used in Reference Example 5, an absorbent material for a test was obtained.

Comparative Example 5

According to the same manner as that of Reference Example 5 except that the water-absorbent resin particle after a particle collision test of Comparative Example 1 was used in Reference Example 5, an absorbent material for a test was obtained.

Example 7

Using 12 g of the water-absorbent resin particle after a particle collision test of Example 3 and 8 g of a ground pulp (Rayfloc manufactured by Rayoneir), they were uniformly mixed by air sheet making to make a sheet-like absorbent material core of a size of 42 cm $\times$ 12 cm.

Then, an upper side and a lower side of the absorbent material were compressed using a roll press in the state where they were held with a tissue paper having a basis weight of 16 g/m$^2$, to make an absorbent material having a density of 0.4 g/cm$^3$.

Further, a top sheet of a polyethylene non-woven fabric (manufactured by Rengo Co., Ltd.) having a basis weight of 22 g/m$^2$ was placed on an upper side of the absorbent material, which was used as an absorbent material for a test.

Example 8

According to the same manner as that of Example 7 except that the water-absorbent resin particle after a particle collision test of Example 4 was used in Example 7, an absorbent material for a test was obtained.

Comparative Example 6

According to the same manner as that of Example 7 except that the water-absorbent resin particle after a particle collision test of Comparative Example 2 was used in Example 7, an absorbent material for a test was obtained.

Absorbent materials for a test obtained in each Example and each Comparative Example were evaluated by the following methods. Results are shown in Table 3.

(8) Absorbent material density

An absorbent material density was calculated from a mass and a thickness of an absorbent material by the following calculation equation.

A thickness of the absorbent material was measured using a thickness meter (PEACOCK J-B manufactured by Ozaki Mfg Co., Ltd.).

$$\text{Absorbent material density (g/cm}^3\text{)} = \text{absorbent material}$$

$$\text{mass (g)/(absorbent material area (cm}^2\text{)} \times \text{thickness (cm))}$$

(9) Leakage test on 45 degree tilting table

An absorbent material for a test is applied to a 45 degree tilting table, and 70 ml of an artificial urine is added dropwise with a burette disposed 2 cm upper from the absorbent material for a test for 10 seconds, at a place which is center between right and left, 10 cm from an upper end of the applied absorbent material for a test. A stopwatch was started at the same time with addition and, after 10 minutes, 70 ml of an artificial urine is added dropwise again with the burette. This procedure was repeated, and an amount of liquid absorption until a test liquid is leaked from a lower end was measured.

Table 3

| Amount of liquid absorption in absorbent material liquid leakage test (ml) | | | | | | |
|---|---|---|---|---|---|---|
| Test liquid injection time | First | Second | Third | Fourth | Fifth | Sixth |
| Example 5* | 70 | 140 | 210 | 280 | 335 | - |
| Example 6 | 70 | 140 | 210 | 280 | 345 | - |
| Comparative Example 5 | 70 | 140 | 210 | 265 | - | - |
| Example 7 | 70 | 140 | 210 | 280 | 350 | 405 |
| Example 8 | 70 | 140 | 210 | 280 | 340 | - |
| Comparative Example 6 | 70 | 140 | 210 | 275 | - | - |
| *Reference example | | | | | | |

From results shown in Table 3, it is seen that all of absorbent materials obtained in respective Examples exert the excellent absorption capacity.

Industrial Applicability

According to the present invention, reduction in water absorbing performance due to collision of an absorbent resin at preparation of an absorbent material is small, and the resulting absorbent product is also excellent in absorbability under pressure, and the present invention can be suitably used in an absorbent material of hygiene materials such as a disposable diaper, a sanitary product and the like.

**Claims**

1. A method for the production of water-absorbent resin particles comprising:

   polymerizing a water-soluble ethylenically unsaturated monomer using a water-soluble radical polymerization initiator, if necessary, in the presence of a crosslinking agent, to obtain water-absorbent resin particle precursors; adding a post-crosslinking agent to crosslink a surface layer of the particle precursors; adding amorphous silica particles in an amount of 0.01 to 2 parts by mass per 100 parts by mass of water-absorbent resin particles; and adjusting the moisture content of the resulting water-absorbent resin particles to 12 to 20%;

   whereby the obtained water-absorbent resin particles have a retaining rate of particle diameter after a particle collision test of 90% or more.

2. The method of claim 1, wherein the moisture content is adjusted to 13 to 20%.

3. Water-absorbent resin particles as obtainable by the method of claim 1 or claim 2.

**4.** The water-absorbent resin particles according to claim 3 wherein the retaining rate of water absorption capacity under pressure after a particle collision test is 60% or more.

**5.** An absorbent material consisting of the water-absorbent resin particles of claim 3 or claim 4, a hydrophilic fiber and a water-permeable sheet.

**6.** The absorbent material according to claim 5, wherein a density of the absorbent material is 0.1 to 0.5 g/cm$^3$.

**7.** The absorbent material according to claim 5 or 6, wherein the ratio of the water-absorbent resin particles and the hydrophilic fiber in the absorbent material is a mass ratio of 30:70 to 80:20.

**Patentansprüche**

**1.** Ein Verfahren zur Herstellung von wasserabsorbierenden Harzteilchen umfassend:

Polymerisieren eines wasserlöslichen ethylenisch ungesättigten Monomers mittels eines wasserlöslichen radikalischen Polymerisationinitiators, gegebenenfalls in Gegenwart eines Vernetzungsmittels, um wasserabsorbierende Harzteilchenvorläufer zu erhalten;
Hinzufügen eines Nachvernetzungsmittels um eine Oberflächenschicht der Teilchenvorläufer zu vernetzen;
Hinzufügen amorpher Siliciumoxidteilchen in einer Menge von 0,01 bis 2 Massenteilen je 100 Massenteilen der wasserabsorbierenden Harzteilchen; und
Einstellen des Feuchtigkeitsgehalts der resultierenden wasserabsorbierenden Harzteilchen auf 12 bis 20 %;
wodurch die erhaltenen wasserabsorbierenden Harzteilchen einen Beibehaltungsanteil der Teilchendurchmesser nach einem Teilchenkollisionstest von 90% oder mehr besitzen.

**2.** Das Verfahren nach Anspruch 1, wobei der Feuchtigkeitsgehalt auf 13 bis 20 % eingestellt wird.

**3.** Wasserabsorbierende Harzteilchen, die durch das Verfahren nach Anspruch 1 oder Anspruch 2 erhältlich sind.

**4.** Die wasserabsorbierenden Harzteilchen gemäß Anspruch 3, wobei der Beibehaltungsanteil der Wasserabsorptionskapazität unter Druck nach einem Teilchenkollisionstest 60 % oder mehr ist.

**5.** Ein absorbierendes Material bestehend aus den wasserabsorbierenden Harzteilchen nach Anspruch 3 oder Anspruch 4, einer hydrophilen Faser und einem wasserdurchlässigen Bahnenmaterial.

**6.** Das absorbierende Material gemäß Anspruch 5, wobei eine Dichte des Absorptionsmaterials 0,1 bis 0,5 g/cm$^3$ beträgt.

**7.** Das absorbierende Material gemäß Anspruch 5 oder 6, wobei das Verhältnis von wasserabsorbierenden Harzteilchen und der hydrophilen Faser in dem absorbierenden Material ein Massenverhältnis von 30:70 bis 80:20 hat.

**Revendications**

**1.** Procédé de production de particules de résine absorbant l'eau comprenant :

la polymérisation d'un monomère à insaturation éthylénique soluble dans l'eau en utilisant un initiateur de polymérisation radicalaire soluble dans l'eau, si nécessaire, en présence d'un agent de réticulation, pour obtenir des précurseurs de particules de résine absorbant l'eau ;
l'ajout d'un agent de post-réticulation pour réticuler une couche de surface des précurseurs de particules ;
l'ajout de particules de silice amorphe en une quantité de 0,01 à 2 parties en masse pour 100 parties en masse des particules de résine absorbant l'eau ; et
l'ajustement de la teneur en humidité des particules de résine absorbant l'eau résultantes à 12 à 20 % ;
les particules de résine absorbant l'eau obtenues ayant un taux de rétention de diamètre de particule après un test de collision de particules supérieur ou égal à 90 %.

**2.** Procédé selon la revendication 1, dans lequel la teneur en humidité est ajustée à 13 à 20 %.

**3.** Particules de résine absorbant l'eau telles que pouvant être obtenues par le procédé selon la revendication 1 ou la revendication 2.

**4.** Particules de résine absorbant l'eau selon la revendication 3, dans lesquelles le taux de rétention de la capacité d'absorption d'eau sous pression après un test de collision de particules est supérieur ou égal à 60 %.

**5.** Matériau absorbant se composant des particules de résine absorbant l'eau selon la revendication 3 ou la revendication 4, d'une fibre hydrophile et d'une feuille perméable à l'eau.

**6.** Matériau absorbant selon la revendication 5, dans lequel une densité du matériau absorbant est de 0,1 à 0,5 g/cm$^3$.

**7.** Matériau absorbant selon la revendication 5 ou 6, dans lequel le rapport des particules de résine absorbant l'eau et de la fibre hydrophile dans le matériau absorbant est un rapport en masse de 30:70 à 80:20.

# Fig. 1

# Fig. 2

**EP 2 048 184 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9124879 A
- EP 1457541 A
- WO 2006062253 A